# EUROPÄISCHE PATENTANMELDUNG

(11) **EP 1 316 289 A1**
(43) Veröffentlichungstag der Anmeldung: **04.06.2003**
(21) Anmeldenummer: 02021143.9
(22) Anmeldetag: 23.09.2002
(51) Int. Cl.: A61B 5/15

(54) **Blutentnahme-Vorrichtung**

(30) Priorität: 22.11.2001 DE 10157171
(71) Anmelder: Smiths Medical Deutschland GmbH, 85614 Kirchseeon (DE)
(72) Erfinder: Glowka, Hermann, 85567 Grafing (DE)
(74) Vertreter: von Bülow, Tam, Dr.

(57) **Zusammenfassung**

Die Blutentnahme-Vorrichtung mit einer als Zwischenspeicher für verdünntes Blut dienenden Kolben-/Zylinderanordnung hat einen im Inneren des Gehäuses (1) verschieblichen Kolben (5), dessen Kolbenstange (7) ein Gewinde (8) aufweist, das mit einer Gewindehülse (12), die ein Innengewinde (13) und ein Außengewinde (14) aufweist, zusammenwirkt, wobei diese Gewindehülse (12) mit einer weiteren Gewindehülse (10) eines Drehgriffes (9) zusammenwirkt. Die Gewindehülsen und die Kolbenstangen sind somit teleskopartig ineinander greifend (Fig. 2).

## Beschreibung

Die Erfindung bezieht sich auf eine Blutentnahme-Vorrichtung gemäß dem Oberbegriff des Patentanspruches 1. Eine derartige Vorrichtung ist aus der DE 42 20 309 C1 bekannt. Diese Blutentnahme-Vorrichtung hat ein Gehäuse, das durch einen drehbaren Deckel verschlossen ist, der wiederum über eine Dichtung das Gehäuse vollständig abdichtet. Der Deckel weist eine Gewindehülse auf, die mit einer durch eine Verdrehsicherung gegen Verdrehen gesicherte Kolbenstange zusammenwirkt, zur Axialverschiebung des Kolbens. Die Kolbenstange hat dabei ein Gewinde, das mit der Gewindehülse zusammenwirkt.

Ein Vorteil dieser bekannten Vorrichtung liegt darin, daß die Kolbenstange in allen Betriebsstellungen des Kolbens innerhalb des Gehäuses bleibt, so daß keine Keime in das Innere des Gehäuses gelangen können. Zur notwendigen Entlüftung hat das Gehäuse eine durch einen Sterilfilter verschlossene Öffnung.

Die US 4,673,386 zeigt eine Blutentnahme-Vorrichtung mit einem Gehäuse und einem darin verschieblichen Kolben, dessen Kolbenstange durch eine Öffnung des Gehäuses herausragt. Theoretisch ist es hierbei möglich, das Volumen des Gehäuses beliebig groß zu machen, was aber den Nachteil bringt, daß die Kolbenstange entsprechend lang wird. Neben dem Nachteil, daß durch die Kolbenstange Keime in das Innere des Gehäuses gelangen können, tritt als weiterer Nachteil auf, daß die Bauhöhe durch die aus dem Gehäuse herausragende Kolbenstange groß wird.

Aufgabe der Erfindung ist es, die eingangs genannte Blutentnahme-Vorrichtung dahingehend zu verbessern, daß das Verhältnis zwischen Bauhöhe und Volumen klein ist und trotzdem die Kolbenstange stets innerhalb des Gehäuses bleibt.

Diese Aufgabe wird durch die im Patentanspruch 1 angegebenen Merkmale gelöst. Vorteilhafte Ausgestaltungen und Weiterbildungen der Erfindung sind den Unteransprüchen zu entnehmen.

Das Grundprinzip der Erfindung liegt darin, die Kolbenstange und die mit dem Drehgriff verbundene Gewindehülse durch eine zweite Gewindehülse mit Innen- und Außengewinde teleskopartig miteinander zu koppeln. Beim Drehen des Drehgriffes verschieben sich die erste Gewindehülse, die zweite Gewindehülse und die Kolbenstange jeweils relativ zueinander. Der gesamte Verschiebeweg des Kolbens entspricht damit in etwa der Summe aus Länge der Kolbenstange und der zweiten Gewindehülse. Die Kolbenstange kann damit also wesentlich kürzer sein als die Verschiebelänge des Kolbens und trotzdem bleibt die Kolbenstange stets vollständig innerhalb des Gehäuses.

Vorzugsweise haben die Kolbenstange, die erste und die zweite Gewindehülse in etwa die gleiche axiale Länge. Hierdurch wird erreicht, daß bei geringster axialer Länge der Blutentnahmevorrichtung der maximale Verschiebeweg des Kolbens ermöglicht wird. Selbstverständlich sind die Länge der Kolbenstange und der ersten und zweiten Gewindehülse so festgelegt, daß sie nicht auseinanderfahren können, sich also voneinander nicht lösen können, wenn der Kolben am ausgefahrenen Anschlag ist.

Im folgenden wird die Erfindung anhand von Ausführungsbeispielen im Zusammenhang mit der Zeichnung ausführlicher erläutert. Es zeigt:
- Fig. 1: einen Querschnitt einer Blutentnahme-Vorrichtung nach einem ersten Ausführungsbeispiel der Erfindung im vollständig ausgefahrenen Zustand des Kolbens;
- Fig. 2 bis 4: ein weiteres Ausführungsbeispiel der Blutentnahme-Vorrichtung in vollständig eingefahrener Stellung, einer Mittelstellung und vollständig ausgefahrener Stellung des Kolbens.

Gleiche Bezugszeichen in den einzelnen Figuren bezeichnen gleiche bzw. funktionell einander entsprechende Teile.

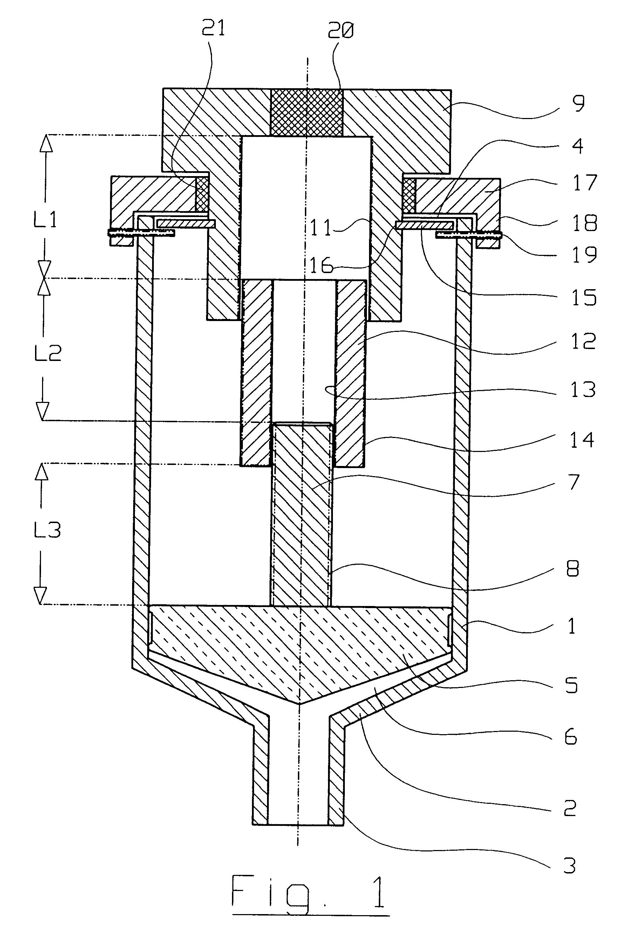

Zunächst wird auf Fig. 1 Bezug genommen. Die Blutentnahme-Vorrichtung hat ein im wesentlichen zylindrisches Gehäuse 1 mit einem konischen Ende 2, das in einen Anschluß 3 mündet, der die Einlaß- und Auslaßöffnung für das Innere des Gehäuses bildet und vorzugsweise als Luer-Anschluß ausgebildet ist. Das gegenüberliegende Ende des Gehäuses 1 hat eine Öffnung 4, deren Durchmesser den Innendurchmesser des Gehäuses 1 entspricht. Im zusammengebauten Zustand ist im Inneren des Gehäuses ein axial verschieblicher Kolben 5 angeordnet, der an seiner zum Anschluß 3 zugewandten Seite eine Konusfläche hat, wobei der Konuswinkel des Kolbens in an sich bekannter Weise (vgl. DE 42 20 301 C1) einen größeren Konuswinkel hat als das konusförmige Ende des Gehäuses, wodurch erreicht wird, daß der Kolben beim Entleeren des Gehäuses die Konusfläche 2 des Gehäuses von außen nach innen kontaktiert und damit vollständig entleert. Der Kolben ist aus elastischem Material.

Am Kolben ist eine Kolbenstange 7 angebracht, die ein Gewinde 8 besitzt, das in diesem Ausführungsbeispiel ein Außengewinde ist. An dem der Öffnung 4 nahegelegenen Ende des Gehäuses 1 ist ein Drehgriff 9 angebracht, der mit einer Gewindehülse 10 durch die Öffnung 4 hindurch in das Innere des Gehäuses ragt. Die Gewindehülse 10 hat in diesem Ausführungsbeispiel ein Innengewinde 11. Weiter ist eine zweite Gewindehülse 12 vorgesehen, die ein Außengewinde 13 und ein Innengewinde 14 hat. Das Außengewinde 13 ist in das Innengewinde 11 der ersten Gewindehülse 10 einschraubbar. Das Innengewinde 14 der zweiten Gewindehülse 12 arbeitet mit dem Außengewinde 8 der Kolbenstange 7 zusammen. Alle genannten Gewinde 11, 12, 13 und 8 haben gleichsinnige Gewinde, also beispielsweise Rechtsgewinde. Vorzugsweise sind alle genannten Gewindeteile aus Kunststoff mit relativ geringer Reibung. Wird ausgehend von der vollständig ausgefahrenen Stellung des Kolben der Fig. 1 der Drehgriff gedreht, so wird die zweite Gewindehülse 12 in die erste Gewindehülse 10 eingeschraubt und nimmt damit auch die Kolbenstange 7 mit dem Kolben 5 mit. Ist die zweite Gewindehülse 12 vollständig in die erste Gewindehülse 10 eingeschraubt, so wird beim weiteren Drehen die Kolbestange 7 in die zweite Gewindehülse eingeschraubt, womit der Kolben weiter nach oben (in Fig. 1) gezogen wird. Aufgrund der relativ größeren Reibungskraft zwischen Außenumfang des Kolbens im Verhältnis zur Reibungskraft der Gewinde ist auch eine Verdrehsicherung für den Kolben vorgesehen. Bei Bedarf kann aber zwischen dem Kolben 5 und der Innenwandung des Gehäuses 1 auch eine zusätzliche Verdrehsicherung vorgesehen sein, beispielsweise durch eine im Inneren des Gehäuses angebrachte axial verlaufende Leiste und eine entsprechende Nut am Außenumfang des Kolbens.

Der Drehgriff 9 ist in einem Deckel 17 drehbar gelagert, der mit einem Überwurfrand 18 das obere Ende des Gehäuses 1 übergreift und mit Schrauben 19 drehfest mit dem Gehäuse 1 verbunden ist. Der Deckel 17 hat eine Öffnung mit einer Dichtung 21, durch welche die Gewindehülse 10 hindurchragt. Die Dichtung 21 verhindert das Eindringen von Luft, Keimen oder sonstigen Verschmutzungen in das Innere des Gehäuses.

Am Außenumfang der ersten Gewindehülse 10 ist im montierten Zustand unterhalb des Deckels eine Nut 16 vorgesehen, in die eine Klemmscheibe 15 eingreift, die zu Montagezwecken einen nicht dargestellten radialen Schlitz hat. Diese Klemmscheibe kann sich je nach Aufwärts- oder Abwärtsbewegung des Kolbens an der Unterseite des Deckels 17 oder den Schrauben 19 abstützen, so daß der Drehgriff 9 mit Gewindehülse 10 in axialer Richtung fixiert ist. Damit beim Verschieben des Kolbens Luft in das Gehäuse einströmen bzw. aus ihm ausströmen kann, hat der Drehgriff 9 ein Sterilfilter 20, das zwar Luft durchläßt, Keime, Staubpartikel oder sonstige Verunreinigungen jedoch nicht ins Innere des Gehäuses läßt.

Aus Fig. 1 ist weiter zu erkennen, daß die Verschiebelängen L1 der zweiten Gewindehülse zur ersten Gewindehülse 10, die Länge L2 und L3, d.h. die Verschiebelänge der Kolbenstange relativ zur zweiten Gewindehülse jeweils gleich groß sind.

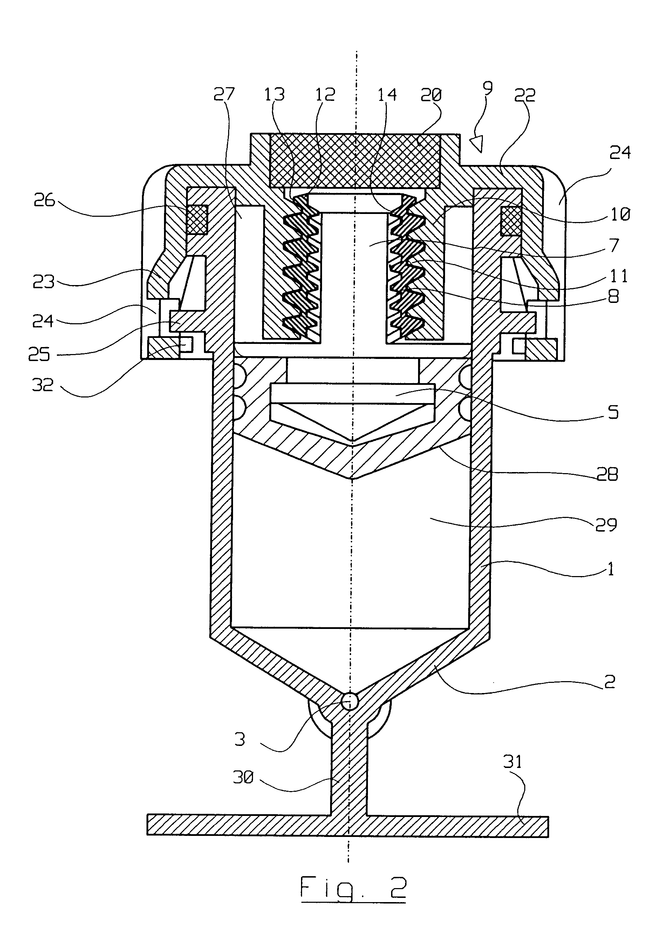

Das Ausführungsbeispiel der Fig. 2 bis 4 unterscheidet sich von dem der Fig. 1 im wesentlichen durch die Konstruktion des Drehgriffes, der hier gleichzeitig die Funktion des Deckels übernimmt. Der Drehgriff 9 hat einerseits die axial verlaufende Gewindehülse 10 mit dem Gewinde 11 sowie einen horizontalen ebenen Abschnitt 22, der die Funktion des Deckels übernimmt sowie einen daran anschließenden, im wesentlichen zylindrischen Abschnitt 23, der die Funktion des Drehgriffes erfüllt und mehrere radial vorstehende Rippen 24 aufweist. Dieser Abschnitt 23 übergreift die Außenseite des Gehäuses 1 und ist gegenüber diesem durch eine Öffnung 24 und einen Vorsprung 34, der mit einem radial vom Gehäuse abstehenden Bund 25 zusammenwirkt, verriegelt, so daß der Drehgriff 9 insgesamt in axialer Richtung gegenüber dem Gehäuse unverschieblich gehalten ist. Zwischen dem Abschnitt 23 und der Außenseite des Gehäuses ist eine Dichtung 26 angeordnet, so daß das Innere des Gehäuses dicht abgeschlossen ist. Im horizontalen Abschnitt 22 ist allerdings zur Entlüftung des Gehäuseinneren 27 ebenfalls der Sterilfilter 20 angeordnet.

Der Kolben 5 hat eine Kolbendichtung 28, die den Kolben manschettenartig umgibt und dichtend an der Innenwandung des Gehäuses 1 anliegt, wodurch das Innere des Gehäuses in zwei Kammern 27 und 29 unterteilt wird. Die untere Kammer 29 ist die Kammer, die mit Flüssigkeit gefüllt wird, während die obere Kammer 27 als Entlüftungskammer bezeichnet wird. In Fig. 2 ist der Kolben 5 in seiner obersten Stellung, d.h. die Gewindehülse 12 ist vollständig eingeschraubt und ebenso die Kolbenstange.

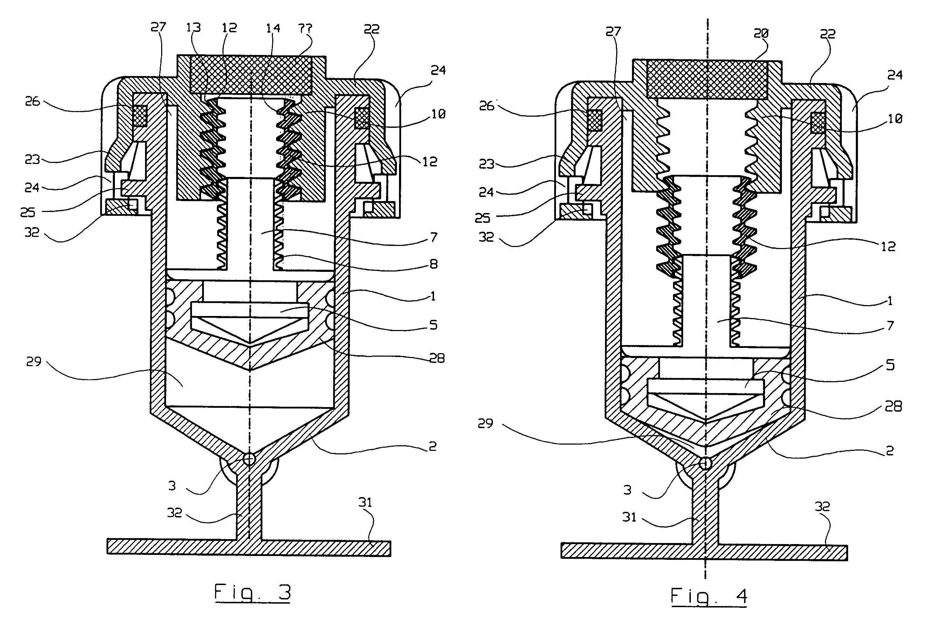

In Fig. 3 ist der Kolben in einer Mittelstellung, in der die Kolbenstange 7 weitestgehend aus der Gewindehülse 12 herausgeschraubt ist, letztere jedoch noch vollständig in der ersten Gewindehülse 10 eingeschraubt ist. In Fig. 4 ist der Kolben in seiner vollständig ausgefahrenen Stellung, bei der alle Gewinde weitestgehend ausgeschraubt sind.

Im Ausführungsbeispiel der Fig. 2 bis 4 ist der Anschluß 3 rohrförmig ausgebildet, so daß die Blutentnahme-Vorrichtung zwischen zwei Schlauchabschnitte angeschlossen werden kann. Am unteren Ende des Gehäuses 1 sind zwei T-förmige Stege 30 und 31 angebracht, damit das Gehäuse 1 an einer Halterung befestigt werden kann.

## Patentansprüche

1. Blutentnahme-Vorrichtung mit einer als Zwischenspeicher für verdünntes Blut dienenden Kolben-/Zylinderanordnung mit zylindrischem Gehäuse und einem im Inneren des Gehäuses verschieblichen Kolben, dessen Kolbenstange ein Gewinde aufweist, das mit einer Gewindehülse eines Drehgriffes zusammenwirkt, **dadurch gekennzeichnet, daß** die Gewindehülse (10) und die Kolbenstange (7) durch eine zweite Gewindehülse (12) mit Außengewinde (13) und Innengewinde (14) miteinander gekoppelt sind.

2. Blutentnahme-Vorrichtung nach Anspruch 1, **dadurch gekennzeichnet,**
**daß** die Verschiebelänge (L1, L2, L3) der beiden Gewindehülsen (10, 12) und der Kolbenstange (7) gleich groß sind.

3. Blutentnahme-Vorrichtung nach Anspruch 1 oder 2, **dadurch gekennzeichnet,**
**daß** der Drehgriff (9) mit Gewindehülse (10) in Axialrichtung gegenüber dem Gehäuse (1) unverschieblich fixiert ist.

4. Blutentnahme-Vorrichtung nach einem der Ansprüche 1 bis 3, **dadurch gekennzeichnet,**
**daß** die Gewindehülsen (10, 11) und die Kolbenstange (7) aus reibungsarmem Kunststoff bestehen.
